# EUROPEAN PATENT SPECIFICATION

(11) **EP 1 007 085 B1**
(45) Date of publication and mention of the grant of the patent: **21.11.2001**
(21) Application number: 98932685.5
(22) Date of filing: 26.06.1998
(51) Int. Cl.: A61K 38/27

(54) **USE OF GROWTH HORMONE IN COMPOSITIONS FOR TREATING INSULIN RESISTANCE IN THE HEART**
VERWENDUNG DES WACHSTUMSHORMONS IN MITTELN ZUR BEHANDLUNG DER INSULINRESISTENZ IM HERZEN
UTILISATION D'HORMONE DE CROISSANCE DANS DES COMPOSITIONS TRAITANT LA RESISTANCE DU COEUR A L'INSULINE

(30) Priority: 04.07.1997 SE 9702595; 08.06.1998 SE 9802023
(43) Date of publication of application: 14.06.2000
(73) Proprietor: Pharmacia Aktiebolag, 112 87 Stockholm (SE)
(72) Inventor: OLIN, Thomas, S-187 44 Täby (SE); REUTERDAHL, Christina, S-178 38 Ekerö (SE); STAHLBOM, Axel, S-129 36 Hägersten (SE); WIELBURSKI, Antek, S-127 33 Skärholmen (SE); RÖNNHOLM, Harriet, S-142 61 Trangsund (SE); JAMES, Stephen, S-162 45 Vällingby (SE)
(74) Representative: Tannerfeldt, Sigrid Agneta
(86) International application number: SE9801261
(87) International publication number: WO9901151

(56) References cited:
- WO-A-95/28173
- WO-A-97/09060
- WO-A-97/38709

## Description

### Field of invention

The present invention relates to the use of growth hormone in the preparation of a therapeutic agent for improving cellular function in the heart challenged by insulin resistance and thereby treating or protecting the heart from complications derivable from this condition.

### Background of the invention

It is widely acknowledged that patients suffering from diabetes mellitus are at risk of acquiring heart disease, see Cardiovasc. Res., 1997, Vol. 34, pp. 1-2. However, in addition to diabetic patients, insulin resistance is also apparent in the heart challenged by stress, extending the need for a treatment of insulin resistance in the heart to patients suffering from both acute and chronic heart failure. Insulin resistance will diminish intracellular signaling mandatory for preserving normal cellular function examplified by intact glucose uptake and metabolism, protection against mediators of cell death (e.g. apoptosis) and cardiac myocyte differentiation which is necessary for proper contractile function (see Cardiovascular Research, Vol. 34(1) pp. 3-55, 1997). Accordingly, insulin resistance in the heart can precipitate heart failure by itself or predispose for serious complication in the stressed heart, which may follow traumatic events such as acute coronary events, congestive heart failure and coronary surgery. Normally, the insulin resistance is treated by applying supra-physiological levels of circulating insulin, by enhancers of insulin action or by dietary restrictions. Such therapies have severe draw-backs or limitations in efficacy with regards to insulin resistance in general. An increased concentration of circulating insulin can cause hyper-proliferation in vascular cells and thereby disturb tissue blood supply. Moreover, insulin enhancers tend to increase body fat mass which in turn is a risk factor in heart disease and dietary restriction is primarily targeted for uncomplicated type 2 diabetes. Further, there is as yet no therapy which has been proven to be efficient in the treatment of complications resulting from insulin resistance in the heart.

The mechanistic basis by which insulin regulates the disposition of glucose by animals has been elucidated in close detail in recent years. A cascade of interacting proteins has been described which, when functioning in the normal situation, serve to transduce the signals emanating from insulin, causing cells of different origin to take up glucose from the bloodstream and store it ( White, M. F. (1997) Diabetologia 40: S2-S17). In the current understanding, activation of the insulin receptor by insulin causes the phosphorylation and activation of insulin receptor substrate (IRS) proteins. These serve to act as docking protein for a variety of downstream protein leading to their activation. A key downstream protein in insulin signalling is phosphoinositide 3-kinase (PI3K) which catalyses the production of the second messenger phosphatidylinositol 3,4,5-trisphosphate. This is a lipid and which is central to the activation of PKB (Franke, T. F et al. (1995) Cell 81: 727-736; James, S. R. et al. (1996) Biochemical Journal 315: 709-713; Franke, T. F et al. (1997) Science 275: 665-668; Klippel, A. et al. (1997) Molecular and Cellular Biology 17: 338-344; Alessi, D. R et al. (1997) Current Biology 7: 261-269; Stokoe, D. et al. (1997) Science 277: 567-570). It is bound by the pleckstrin homology (PH) domains of PKB and of an upstream kinase called 3-phosphoinositide-dependent kinase 1 (PDKI) which is involved in the activation of PKB.

It is also known that PKB inhibits BAD (a member of the BCL2 protein family) by phosphorylation, thereby preventing the apoptotic promoting effect of BAD (see del-Peso-L et al, Science, 1997, 278 (5338):687-9; Datta et al, Cell, 1997;91(2):231-41).

PKB appears to be a key intermediary in the regulation of glucose utilisation and control of protein synthesis by insulin (Cross, D., et al. (1995). Nature 378: 785-789; Cohen, Pet al. (1997). FEBS Letters 410: 3-10; Peak, M., J. et al. (1998). Diabetologia 41: 16-25)Gingras, A.-Cet al. (1998). Genes and Development 12: 502-513). Thus, it has been demonstrated to phosphorylate and inactivate glycogen synthase kinase 3 (GSK3); permitting the synthesis of glycogen from glucose. Furthermore, in cardiac myocytes, PKB has been shown to phosphorylate and activate phosphofructo kinase-2 (Deprez, D et al. (1997) Journal of Biological Chemistry 272: 17269-17275) whose product, fructose 2,6-bisphosphate, acts as an allosteric activator of glycolysis. A third likely substrate for PKB is the type 3B cyclic AMP phosphodiesterase ( Wijkander, J. et al. (1998). Endocrinology 139: 219-227), which in insulin-responsive tissues is activated by phosphorylation, leading to the inactivation of adrenergic-stimulated processes.

It is hence, highly desirable to find a therapy with which the cardiac cellular function, otherwise impaired by insulin resistance, can be retained in a normal state or adjusted into improved function. In particular, it would be advantageous in such a therapy to employ a native substance with capacity to overcome resistance in the insulin signal transduction pathway and thereby protect the heart from further damage. Such therapy applies to diabetic patients at risk of developing cardiac failure, chronic heart failure patients with apparent insulin resistance and patients suffering from an acute cardiac trauma who are often is characterized by an inability to respond to insulin.

Human growth hormone is a native signaling substance which is reported to be of potential benefit in several aspects of cardiac therapy. For example, the International Patent Application WO 9528173 to Genentech Inc. discloses successful treatments of congestive heart failure with human growth hormone resulting in improved cardiac function. In contrast, there are reports of non-responding patients to human growth hormone treatment of heart-failure, for example Frustaci A et al., N Engl J Med 335(9): 672-4, 1996. Human growth hormone has also been demonstrated to exert a positive effect on cardiac insufficiency by supporting an adequate growth of cardiac tissue after dilation of the ventricle in idiopathic cardiomyopathy (Fazio S et al., N EngI J Med 334:809-14, 1996). Furthermore, human growth hormone is reported to suppress cell death (apoptosis) in several cell types, see e.g. Biol. Reprod., 1995, Vol. 53(1), pp. 13-20 (K Eisenhauer et al.) and Horm. Res., 1996, Vol. 46 (4-5), pp. 215-221 (S Hirschfeldt).

Growth hormone replacement supplementation to hypophysectomized rats has been shown to improve glucose uptake in the diaphragm muscle, see Diabetes, 1962, Vol. 11(3), pp. 171-178. By contrast, pharmacological treatment with GH, resulting in supra-physiological circulating levels of the hormone, is known to produce a decrease in total body glucose uptake and disposal as well as a blunted metabolic response to insulin (i.e. insulin resistance) in skeletal muscles, see Hettiarachchi M et al., Diabetes 45(4):415-21, 1996. However, in tissues which are metabolically markedly different from the muscle, like adipose tissue, GH could induce glucose up-take, as reported in Endocrinology, 1996, Vol. 137(11), pp. 4650-5 M Ridderstråle et al.

It is therefore unexpected that growth hormone therapy could be used to induce an insulin-like signaling in cardiac muscle cells producing an improved glucose metabolism and tolerance to stress, in the absence of insulin.

### Description of the invention

The present invention is based on the finding that human growth hormone can exert insulin-like effects in cardiac cells and thereby is capable of replacing this hormone during insulin resistance. In general, the present invention relates to the utility of an effective amount of human growth hormone in the preparation of an agent for the administration to a patient, having or being at risk of acquiring insulin resistance in the heart. More particularly, the present invention relates to the use of an effective dose of human growth hormone in the preparation of an agent for retaining an adequate metabolism and resistance to stress in the heart challenged by insulin resistance and to prevent the heart from complications derived from insulin resistance. Typically, the insulin resistance may be induced by chronic stress or traumatic events including surgery, but it may also derive from conditions related to diabetes.

The therapy is specifically targeted to normalize a disturbed cardiac metabolism, including glucose transportation, glucose oxidation and fatty acid oxidation, to achieve a sufficient coronary blood flow or to induce protection against stress-induced cell death in order to maintain an adequate cardiac contractile function which may eventually precipitate as a result of a diminished insulin receptor dependent signaling in the heart. In addition, it has been found that the growth hormone therapy enhances the activity of PKB (Protein Kinase B) which is a key intermediary agent in the regulation of glucose utilization and control of protein synthesis by insulin. Consequently, growth hormone preparations can be used for improving a compromised heart function common in acute failure caused by e.g. myocardial infarction, hypertension, genetic disorders, as well as complications resulting from insulin resistance and thereby treating or protecting the heart.

The use according to the present invention provides a suitable treatment especially for patients suffering from diabetes involving insulin resistance and have, or are at risk of acquiring, cardiac complications. Furthermore, patients suffering from chronic and acute heart failure will benefit from the therapy if they are exposed to conditions, such as stress or trauma from which insulin resistance is developed. The skilled physician will be able to judge which disorders may develop into insulin resistance and thereby further challenge such cardiac patients.

Furthermore, it is also possible to determine whether an individual; who has or is at risk of acquiring cardiac complications, is eligible for a treatment with human growth hormone by subjecting the patient to an insulin resistance test, for example an oral glucose tolerance tests, glucose/clamp analysis or analysis of the fasting blood glucose level. Alternatively, a test allowing for a specific determination of the insulin resistance in the heart is also applicable when suitable to apply, as for example in connection with bypass surgery. Such tests will provide a simple and efficient prognostic diagnostic tool for determining if the patient will benefit from a human growth hormone therapy for treating or preventing the heart from further complications due to insulin resistance.

The utility of human growth hormone will consequently be especially powerful in pathological states where insulin is prevented from exerting its full action (insulin resistance) and where cardiac complications clearly are developing. The cardiac complications associated with insulin resistance can be clinically defined as chronic ischemia, contractile dysfunction or congestion. At the tissue and cellular level, the cardiac insufficiency can be identified by cardiac enzyme leakage into the blood such as cardiac isoforms of creatine kinase and troponin, an inadequate blood perfusion of the heart, a deranged metabolism exemplified by a low glucose up-take and high lactate production, a malfunctioning calcium regulation or an increase in cell death.

"Human growth hormone (hGH)" employed in the preparation of effective compositions for treating insulin resistance and its accompanying complications is intended to have a broad definition in the context of the present invention and its appended claims. It refers to any form of native, recombinant or synthetic polypeptide or protein exhibiting hGH-activity including all variants of hGH with functionally analogue activity, such as truncated forms, fragments thereof, forms with amended or extended amino acid sequence or derivatives of hGH including conjugates with complementary agents for modulating its characteristics. The definition shall also include other compounds with growth hormone activity such as molecules mimicking hGH action having affinity to the hGH receptor and/or capability of activating the hGH receptor.

The agents comprising hGH according to the present invention preferably can be conventional therapeutic formulations of hGH prepared for storage by including one or several optional additives of physiological characteristics (carriers, excipients or stabilizers) to provide lyophilized products or aqueous solutions which are aimed for parenteral administration, either directly, or after reconstitution with a physiological fluid. One such suitable hGH product is Genotropin (as supplied by Pharmacia & Upjohn AB). A number of other formulations of hGH which might be conceivable to use with present invention are discussed in the mentioned publication WO 95/28173. Also other administration forms would be conceivable for the skilled person to employ with the present invention, if they could be considered to provide an effective dose.

The effective amount of hGH necessary to administer to treat the above mentioned complications will vary dependent on the patient and the administration form. The clinically skilled person will be able adjust doses and select the appropriate administration route in order to reach the desired, mentioned therapeutic effects and thereby survey the patients response with conventional methods and tests.

Even if the present invention is mainly directed to the use of human growth hormone for preparing therapeutic agents to treat humans, it is conceivable to extend it therapy of other mammals with growth hormones with corresponding biological activity.

The following exemplifying part of description intends to illustrate the present invention and it is believed that the results are possible to repeat after suitable administration of growth hormone to mammals including humans suffering from insulin resistance.

### Detailed description of the invention

Fig. 1 shows that the cell viability increases in cardiac myocytes after human growth hormone supplementation.

Fig. 2 demonstrates that the transport of glucose increases in cardiac myocytes when stimulated with human growth hormone.

Fig. 3 shows the influence of wortmannin on the glucose transport in cardiac myocytes stimulated by growth hormone.

Fig. 4 shows apoptosis induced with mitomycin and SNAP and the effects obtained with GH and IGF-1 in the presence of and without wortmannin.

Fig. 5 shows how glucose oxidation and palmitate oxidation are affected when cardiac myocytes are stimulated by growth hormone.

Fig. 6 shows how Protein Kinase B (PKB) is activated in cardiac myocytes by growth hormone.

### Example 1

This example aims to investigate whether that cardiac myocytes expresses functionally coupled Growth Hormone receptors and the metabolic effect of the hormone in these cells. Cardiac myocytes used in the current experiment were highly differentiated and prepared from mammals.

Dulbeccos Modified Eagle's medium (DMEM), Penicillin and Streptomycin (PEST), Foetal Calf Serum (FCS) were bought from Gibco Laboratories, USA. Tissue culture supplies were from Costar, USA. rhGH, Genotropin was from Pharmacia & Upjohn. Antibodies against GH receptor (Mab 263, D7.18.3D5/131, batch #174A-005) were purchased from Agen, Australia.

Cardiac myocytes were isolated according to conventional routines for preparation of primary cultures and maintained in Dulbeccos Modified Eagle's medium supplemented with penicillin (100 U/ml), streptomycin (100 µg/ml) and 10 % foetal bovine serum (FCS).

Growth Hormone receptors were detected by Western Blotting method using solubilized membrane preparations or by using Flow Cytometric Analysis (Kamentsky L.A.(1973) Cytology automation, Adv. Biol. Med. Phys. 14:93).

Cell viability was measured by using an assay kit, Ez4u by Biomedica. The method is based on the finding that intact cells are capable to reduce uncolored tetrazolium salts to colored derivatives, which are easily measured spectrophotometrically. This method discriminates between living and dead cells because the reduction process requires functional mitochondria which are inactivated after cell death. Thus, the amount of viable cells are directly proportional to OD.

Glucose transport was measured as described by Hundal et al (1994). Briefly, cells were incubated in serum free medium for 4 hours followed by incubation with Growth Hormone for 60 minutes, if not otherwise stated. Then cell monolayers were rinsed with Krebs Ringer Bicarbonate buffer (KRB) pH 7.4. Glucose transport was quantified by incubating the cells in the presence of ³H-2-deoxy-glucose in KRB for 8 minutes. Non-specific uptake was determined by quantifying cell associated radioactivity in the presence of 10 µM cytochalasin B. Uptake of 2-deoxy-glucose was terminated by rapidly aspirating the radioactive medium, followed by three successive washes of cell monolayers with cold PBS. The cells were lysed in 0.5 M NaOH before liquid scintillation counting. Rates of glucose transport were normalized for protein content in each well.

The membrane proteins were separated by SDS-polyacrylamide gel electrophoresis (8 % acrylamide) according to the method of Laemmli (1970).

Western blotting was performed according to Towbin et al., (1979). Presence of immuno-complexes on the nitrocellulose filters was determined by the ECL method following the description of the manufacturer (Amersham).

### Detection of GH receptors

Cardiac myocytes were harvested according to standard techniques for adherent cells. The samples were separated on SDS-PAGE and transferred onto nitrocellulose membrane. The membrane was incubated in the presence of anti-GH receptor. Antibodies and antigen-antibody complexes were detected by a chemiluminescent method (ECL). The cells were immunostained with a-GHR antibodies and a secondary FITC-conjugated rabbit anti mouse F(ab)2. The analysis was performed with an EPICS XL flow cytometer using an 488 nm argon laser and 525 nm band pass filter. The results show that growth hormone receptors are available on the surfaces on the AT-1 cells. Functional coupling of the receptors were confirmed by activation STAT5, using a gel-shift assay revealing binding of the transcription factor to a specific nucleotide sequence, and the biological response demonstrated below.

### Cell viability

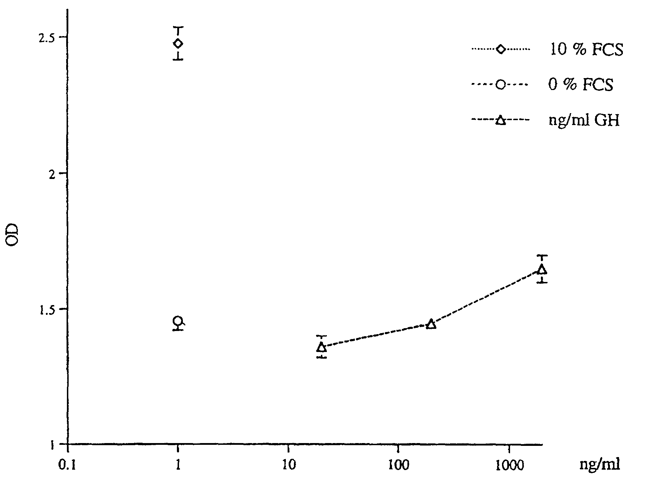

Cells were incubated for 24 hours in serum free medium supplemented with 20, 200 or 2000 ng/ml GH. As control, cells were incubated in serum free medium only or in medium containing 10 % FCS. In serum starved cells a 40 % decrease in viability was seen compared to cells incubated in 10% FCS. As shown in Fig. 1 GH has a beneficial effect of cell survival, the highest dose used, 2000 ng/ml, showed a significant difference in OD, or viable cells, compared to cells incubated in serum free medium only. Further, the viability score shows that these cells were suitable for experimental use.

### Glucose transport

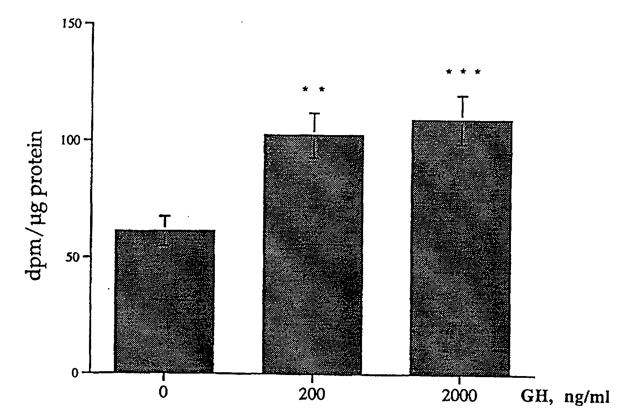

The cardiac myocytes were incubated in serum free medium for 4 hours followed by 60 minutes incubation of GH of different doses in serum starved cells. As demonstrated in Fig. 2, an increase in glucose transport rate was seen. The doses, 200 and 2000 ng/ml, significantly increased the rate by 68 and 78 %, respectively.

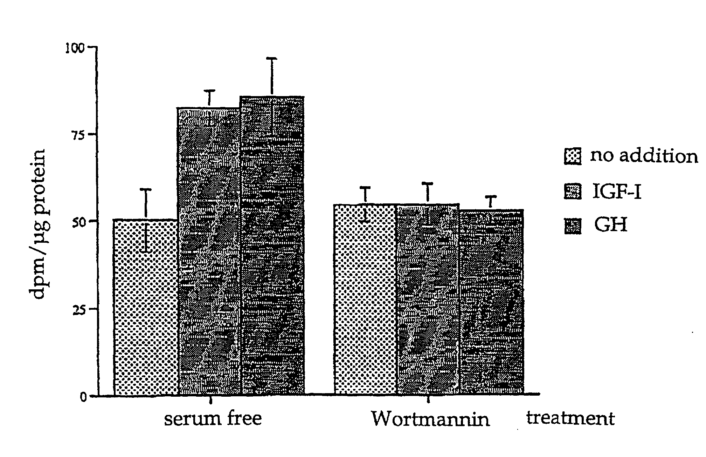

In another assay, the cardiac myocytes were incubated in serum free medium with or without 1 µm wortmannin for 4 hours. During the last hour, the cells were either stimulated with 200 ng/ml of GH or as a reference with 100 ng/ml of IGF-1. Glucose transport measurements were performed and as shown in Fig.3, the wortmannin treated cells does not respond with an increased transportation of glucose. This is due to that wortmannin effectively inhibits the signal transduction generated from the insulin receptor by its specific inactivation the signal transducer phosphoinositide 3-kinase (PI 3-kinase).

### Example 2

Cardiac myocytes were grown and treated in accordance with Example 1.

In order to evaluate the effects of growth hormone on apoptosis in cardiac myocytes, 5 mM of the nitric oxide donor SNAP (S-nitroso-N-acetyl-DL-penicillamine) and 30 µm of the DNA cross-linker and p53 inducer, mitomycin, were added to samples of cardiac myocytes as apoptosis inducers according to the following scheme:

| Bottle | mitomycin | SNAP | additives |
|---|---|---|---|
| 1 | + | + | - |
| 2 | + | - | - |
| 3 | - | + | - |
| 4 | - | - | - |
| 5 | + | + | IGF-1 100 ng/l |
| 6 | + | + | GH 500 ng/l |

30 µm mitomycin and 5 mM SNAP were added to serum free media, whereupon the bottles were incubated for 6 hours and 16 hours, respectively. All cells were incubated overnight before samples were taken and labeled for TUNEL analysis (an end-labeling technique identifying fragmented DNA which is characteristic for programmed cell-death, apoptosis) with following results:

**Table 1**

| Additives | Apoptotic cells (%) |
|---|---|
| Mitomycin + SNAP | 38.9 |
| Mitomycin | 15.2 |
| SNAP | 0.6 |
| none | 0.8 |
| Mitomycin + SNAP + IGF-1 | 4.4 |
| Mitomycin + SNAP + GH | 3.5 |

The results of Table 1 clearly demonstrate that an addition of mitomycin and SNAP induces apoptosis in cardiac myocytes and that the GH is powerfully protecting against cell death (apoptosis).

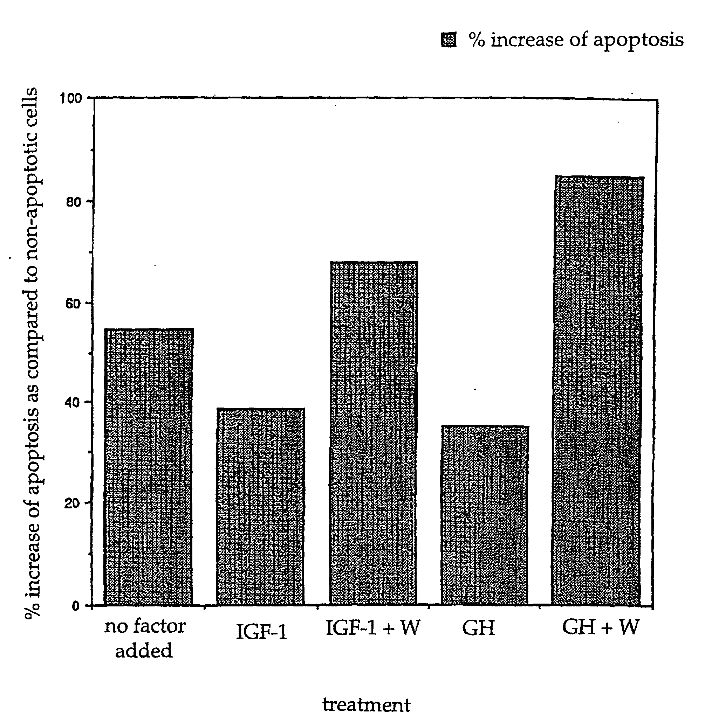

The experiment was repeated with an addition of 0.1 µm wortmannin to foetal cardiac myocytes challenged with, mitomycin in combination with SNAP, and treated with GH or IGF-1 according to the above described experiment . The results demonstrated in Fig. 4 show that wortmannin abolishes the protection against apoptosis exerted by GH and IGF-I. The ability of wortmannin to abolish the protective capacity of GH provides further evidence that the beneficial effects of said hormone is mediated by an insulin-like signaling which involves PI3K.

### Example 3

Cardiac myocytes were grown and treated in accordance with Example 1. Oxidative rates of 14C-glucose or 14C-palmitic acid were determined according to the principle described by Lopaschuk et al (1989) (Gary D. Lopaschuk, Gordon F. McNeil and Jefferey J. McVeigh, 1989, Mol. Cell. Biochem., 88:175-179.)

### Glucose oxidation

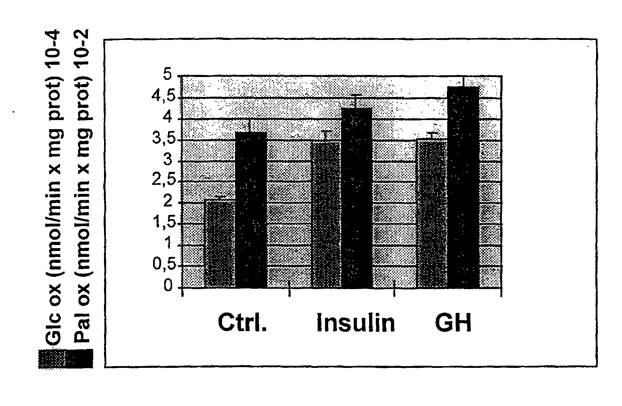

The cardiac myocytes were incubated in serum free medium for 4 hours and subsequently 3 ml medium was supplemented with ¹⁴C-glucose (0.2µCi/ml, 5mM glucose) +/- insulin/GH was added. Devices containing filter paper were mounted in the cultivation flasks, the screw caps were tightened and cells were incubated for additional hour at 37° C. Prior termination of the CO₂ measurements 0.4 ml of hyamine solution was applied on filter paper followed by 0.4 ml of 4 M sulphuric acid injection into the medium. The flasks were then incubated for additional 60 minutes at room temperature on shaker. Finally, the filters were cut into small pieces and transferred into scintillation vials, scintillation solution (10ml) and methanol (200µl) was added to each vial and the samples were counted for radioactivity. As blanks, flasks containing medium (no cells), were processed exactly as described above. Figure 5 shows that GH and insulin stimulates glucose oxidative rate in cardiac myocytes

### Palmitic acid oxidation

The cardiac myocytes were incubated in serum free medium for 4 hours and subsequently 3 ml medium supplemented with ¹⁴C-palmitate(120uM (0.2µCi)/ml) +/-insulin /GH was added to each flask. Devices containing filter paper were mounted in the cultivation flasks, the screw caps were tightened and cells were incubated for 1 hour at 37° C. Prior termination of the CO₂ measurements 0.4 ml of hyamine solution was applied on filter paper followed by injection of 0.4 ml of 4 M sulphuric acid into the medium. The flasks were than incubated for additional 60 min. at room temperature on shaker. Finally the filters were cut into small pieces and transferred into scintillation vials, scintillation solution (10ml) and methanol (200µl) was added to each vial and the samples were counted for radioactivity. As blanks, flasks containing medium (no cells), were processed exactly as described above. Figure 5 shows that GH and insulin stimulates palmitic acid oxidative rate in cardiac myocytes

### Example 4

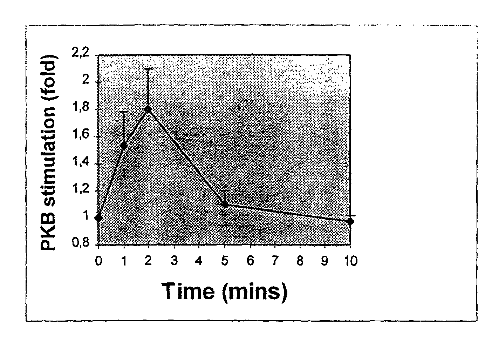

To further elucidate the involvement of PI3K related signaling in the effects exerted by GH on the cardiac myocyte, the activity of protein kinase B (PKB) was assayed. Cardiac myocytes were grown and treated in accordance with Example 1. Thereafter the cells were starved and then lysed and the protein concentration of lysates determined. Lysates were matched for protein and volume, and the a isoform of PKB was immunoprecipitated using specific antibodies pre-coupled to protein G beads. After rigorous washing, immunoprecipitates were assayed for PKB activity for 30 minutes using a specific PKB peptide substrate as previously described (Alessi, D. et al (1997) Current Biology 7, 261-269). Figure 6 shows that GH produces a rapid and pronounced activation of PKB.

### Discussion

In summary, it is demonstrated from the Examples 1 and 2, that the cardiomyocytes express functionally coupled GH receptors. The examples further show that GH exerts both a metabolic and protective effect on the cardiac myocyte, as reflected by a stimulated glucose transport rate and an decreased rate of stress-induced cell death, respectively. The marked increase in cardiac myocyte glucose and fatty acid oxidation in response to GH treatment expands the scope of the present invention to a treatment which enhances the efficiency by which nutrients like glucose and fatty acids are converted to energy. This increased energy supply is anticipated to improve contractile function and efficiency of the heart.

Moreover, it can be concluded that GH activates signal transducers, for example phosphoinositide 3-kinase and activators downstream of this, which are important mediators of the insulin action for sustaining an adequate metabolism and protection against stress-induced cell death. This statement is substantiated by the rapid and prominent activation of PKB following GH treatment. The insulin-like effect exerted by GH is insulin independent and can hence be therapeutically used in situations where the insulin action is inhibited as is the case in insulin resistance appearing in diabetes or certain cases of acute and chronic heart failure. The therapeutic potential of GH treatment of the heart should not be limited to glucose transport and protection against cell death, as these effects are only proof of the principle that GH can act via the insulin signaling pathway. Hence, the GH action on the heart can be extended to further effects of insulin on muscle tissue, for example oxidative capacity, cellular differentiation state and contractile function.

## Claims

1. The use of an effective dose of human growth hormone in the preparation of an agent for the treatment of a patient, having or being at risk of acquiring insulin resistance in the heart.

2. The use according to claim 1, wherein an adequate glucose metabolism is retained and resistance to stress is obtained in the heart challenged by insulin resistance.

3. The use according to claims 1 or 2 **characterized by** preventing the heart from complications derived from insulin resistance.

4. The use according to any of claims 1 to 3, wherein the insulin resistance is induced by stress or traumatic events including surgery.

5. The use according to any of claims 1 to 4, wherein a deranged glucose metabolism of the heart cells is normalized or prevented.

6. The use according to claim 5, wherein glucose oxidation is improved.

7. The use according to claims 1, 3 or 4, wherein the fatty acid oxidation of the heart cell is improved.

8. The use according to any of claims 1 to 7, wherein a sufficient coronary blood flow is improved.

9. The use according to any of claims 1 to 8, wherein the contractile function or efficiency of the heart is improved.

10. The use according to any of claims 1 to 7, wherein the treated patients suffer from diabetes mellitus or from chronic or acute heart failure.

11. The use of an insulin resistance test to provide a diagnostic means for determining if an individual having or being at risk of acquiring cardiac complications, is susceptible for a human growth hormone therapy for treating or preventing the heart from complications derived from insulin resistance.

## Patentansprüche

1. Verwendung einer wirksamen Dosis von menschlichem Wachstumshormon bei der Herstellung eines Mittels für die Behandlung eines Patienten, der Insulinresistenz im Herzen hat, oder bei dem ein Risiko besteht, Insulinresistenz im Herzen zu bekommen.

2. Verwendung nach Anspruch 1, wobei in dem Herzen, das mit Insulinresistenz konfrontiert ist, ein adäquater Glucosemetabolismus aufrecht erhalten und Widerstandsfähigkeit gegen Stress erzielt wird.

3. Verwendung nach den Ansprüchen 1 oder 2, **dadurch gekennzeichnet, dass** das Herz vor Komplikationen, die sich von der Insulinresistenz ableiten, geschützt wird.

4. Verwendung nach einem der Ansprüche 1 bis 3, wobei die Insulinresistenz durch Stress oder traumatische Ereignisse, einschließlich eines chirurgischen Eingriffs, induziert wird.

5. Verwendung nach einem der Ansprüche 1 bis 4, wobei ein gestörter Glucosemetabolismus der Herzzellen normalisiert oder verhindert wird.

6. Verwendung nach Anspruch 5, wobei die Glucoseoxidation verbessert wird.

7. Verwendung nach den Ansprüchen 1, 3 oder 4, wobei die Fettsäureoxidation der Herzzelle verbessert wird.

8. Verwendung nach einem der Ansprüche 1 bis 7, wobei eine ausreichende Koronardurchblutung verbessert wird.

9. Verwendung nach einem der Ansprüche 1 bis 8, wobei die Kontraktionsfunktion oder Effizienz des Herzens verbessert wird.

10. Verwendung nach einem der Ansprüche 1 bis 7, wobei die behandelten Patienten unter Diabetes mellitus oder unter chronischem oder akutem Herzversagen leiden.

11. Verwendung eines Insulinresistenztests, um ein diagnostisches Mittel bereitzustellen, um zu bestimmen, ob ein Individuum, das Herzkomplikationen hat oder bei dem ein Risiko besteht, Herzkomplikationen zu bekommen, empfänglich ist für eine Therapie mit menschlichem Wachstumshormon zum Behandeln des Herzens hinsichtlich oder Schützen des Herzens vor Komplikationen, die sich von Insulinresistenz ableiten.

## Revendications

1. L'utilisation d'une dose efficace d'hormone de croissance humaine pour la préparation d'un agent de traitement de patients dont le coeur présente une résistance acquise à l'insuline ou qui sont à risque pour acquérir une telle résistance.

2. Une utilisation selon la revendication 1 par laquelle un métabolisme glucidique adéquat est préservé et une résistance au stress est obtenue dans un coeur soumis à une résistance à l'insuline.

3. Une utilisation selon les revendications 1 ou 2 **caractérisée par** la prévention de complications cardiaques provenant de la résistance à l'insuline.

4. Une utilisation selon l'une quelconque des revendications 1 à 3 lorsque la résistance à l'insuline est induite par le stress ou par des événements traumatiques, y compris une opération chirurgicale.

5. Une utilisation selon l'une quelconque des revendications 1 à 4 par laquelle les perturbations du métabolisme glucidique des cellules cardiaques sont normalisées ou évitées.

6. Une utilisation selon la revendication 5 par laquelle l'oxydation du glucose est améliorée.

7. Une utilisation selon les revendications 1, 3 ou 4 par laquelle l'oxydation des acides gras dans les cellules cardiaques est améliorée.

8. Une utilisation selon l'une quelconque des revendications 1 à 7 par laquelle le débit sanguin coronarien est amélioré.

9. Une utilisation selon l'une quelconque des revendications 1 à 8 par laquelle la fonction contractile ou l'efficacité du coeur est améliorée.

10. Une utilisation selon l'une quelconque des revendications 1 à 7 dans laquelle les patients traités ont un diabète sucré ou une insuffisance cardiaque chronique ou aiguë.

11. L'utilisation d'un test de résistance à l'insuline pour fournir un moyen de diagnostic permettant de déterminer si un individu ayant des complications cardiaques ou à risque pour l'acquisition de telles complications est susceptible de recevoir un traitement par l'hormone de croissance humaine pour traiter ou prévenir les complications cardiaques dérivées de la résistance à l'insuline.
